# EUROPEAN PATENT APPLICATION

(11) **EP 3 219 205 A1**
(43) Date of publication of application: **20.09.2017**
(21) Application number: 17158072.3
(22) Date of filing: 27.02.2017
(51) Int. Cl.: A01K 67/027

(54) **METHOD FOR PRODUCING A NON HUMAN ANIMAL MODEL OF OSTEOBLASTIC BONE METASTASIS**

(30) Priority: 18.03.2016 JP 2016055736
(71) Applicant: Nihon Medi-Physics Co., Ltd., Tokyo 136-0075 (JP)
(72) Inventor: OKA, Shuntaro, Koto-ku, Tokyo 136-0075 (JP); KANAGAWA, Masaru, Koto-ku, Tokyo 136-0075 (JP); ONO, Masahiro, Koto-ku, Tokyo 136-0075 (JP); DOI, Yoshihiro, Koto-ku, Tokyo 136-0075 (JP)
(74) Representative: TBK

(57) **Abstract**

Provided is a method for producing an animal model of osteoblastic bone metastasis. A non-human animal in which an osteoblastic lesion is formed in a wide range has been successfully produced with a probability of 100% by: administering a calcineurin inhibitor to a non-human animal, and injecting a tumor cell into an artery or a vein of the non-human animal, wherein the non-human animal and the tumor cell are in an allogeneic or xenogeneic relation.

## Description

### [Technical Field]

The present invention relates to a method for producing an animal model of osteoblastic bone metastasis. Moreover, the present invention also relates to a screening method for a compound for detecting an osteoblastic bone metastasis and a screening method for a compound having an activity of suppressing an osteoblastic bone metastasis both of which utilize the production method. Further, the present invention relates to an animal model of osteoblastic bone metastasis produced by the production method.

### [Background Art]

A bone metastasis refers to lesion formation in a bone as a result of tumor cell migration through blood vessels, and is pathologically mainly classified into four types: osteoblastic bone metastasis; osteolytic bone metastasis; mixed bone metastasis, which is a mixture of the two; and inter-trabecular bone metastasis from which neither bone formation nor osteolysis is observed. Meanwhile, the number of effective treatment methods against primary lesions has been increased recently, making it possible to extend the lifetimes of cancer patients. However, bone metastases cause various severe symptoms such as pain, bone fracture, and paralysis, lowering qualities of life of patients and bringing about problems. Particularly, since patients having osteoblastic bone metastases live longer than those having the other bone metastasis types, there is a strong demand for development of a method for treating or preventing the metastasis.

Moreover, recently, in the developments of therapeutic agents and diagnostic agents against cancers, efforts have been made to prepare cancer-bearing animal models to evaluate efficacies, effects, and ADME of such agents. As to osteoblastic bone metastases also, it has been reported that animal models thereof are prepared by injecting tumor cells into the bone marrow cavity (Non Patent Literatures 1 and 2). However, since these animals are prepared by directly injecting tumor cells into bones, the developmental mechanism is different from the original mechanism in which tumor cells migrate through blood vessels and form a lesion in a bone. Hence, the prepared animals are not effective as animal models.

In addition, regarding osteoblastic bone metastases, it has also been reported that an effort was made to prepare animal models by injecting tumor cells into the left ventricle of the heart or into the tail vein (Non Patent Literature 2). However, although hind-leg movement disorders indicative of a bone metastasis were observed from animals obtained by such methods involving blood vessels, osteoblastic lesions which could be detected by scintigraphy were not detected.

### [Citation List]

### [Non Patent Literature]

[NPL 1] Lamoureux F. et al., Int. J. Cancer, 2008, 122 (4), pp. 751 to 760
[NPL 2] Liepe K. et al., Anticancer Res., 2005, 25 (2A), pp. 1067 to 1073

### [Summary of Invention]

### [Technical Problem]

The present invention has been made in view of the above-described problems of the conventional techniques. An object of the present invention is to provide a non-human animal in which tumor cells migrate through a blood vessel and form an osteoblastic lesion in a bone.

### [Solution to Problem]

The present inventors have earnestly studied to achieve the above object. As a result, the inventors have found that administering a calcineurin inhibitor to a rat and then injecting an artery thereof with tumor cells allogeneic in relation to the rat form an osteoblastic lesion in a wide range with quite a high probability.

The present invention is based on the above-described study result, and relates to a method for producing an animal model of osteoblastic bone metastasis. Moreover, the present invention also relates to a screening method for a compound for detecting an osteoblastic bone metastasis, and a screening method for a compound having an activity of suppressing an osteoblastic bone metastasis. Further, the present invention relates to an animal model of osteoblastic bone metastasis produced by the production method. More specifically, the present invention is as follows.
<1> A method for producing an animal model of osteoblastic bone metastasis, the method comprising the steps of:
   administering a calcineurin inhibitor to a non-human animal; and
   injecting a tumor cell into an artery or a vein of the non-human animal, wherein
   the non-human animal and the tumor cell are in an allogeneic or xenogeneic relation.
<2> A screening method for a compound for detecting an osteoblastic bone metastasis, the method comprising the steps of:
   (1) administering a calcineurin inhibitor to a non-human animal;
   (2) injecting a tumor cell into an artery or a vein of the non-human animal to form an osteoblastic lesion;
   (3) providing the non-human animal with a test compound;
   (4) detecting the test compound at a site of the osteoblastic lesion; and
   (5) based on an accumulated amount of the test compound detected at the site of the osteoblastic lesion, judging whether or not an osteoblastic bone metastasis is detectable with the compound, wherein
   the non-human animal and the tumor cell are in an allogeneic or xenogeneic relation.
<3> A screening method for a compound having an activity of suppressing an osteoblastic bone metastasis, the method comprising the steps of:
   (1) administering a calcineurin inhibitor to a non-human animal;
   (2) injecting a tumor cell into an artery or a vein of the non-human animal;
   (3) providing the non-human animal with a test compound before, during, or after the injection of the tumor cell;
   (4) detecting an osteoblastic lesion level in the non-human animal provided with the test compound;
   (5) detecting an osteoblastic lesion level in the non-human animal injected with the tumor cell in the step (2) but not provided with the test compound;
   (6) comparing the osteoblastic lesion levels detected in the steps (4) and (5), and determining that the test compound is a compound having an activity of suppressing an osteoblastic bone metastasis if the osteoblastic lesion level detected in the step (4) is lower than the osteoblastic lesion level detected in the step (5), wherein
   the non-human animal and the tumor cell are in an allogeneic or xenogeneic relation.
<4> The method according to any one of <1> to <3>, wherein the tumor cell is a prostate cancer cell or a breast cancer cell.
<5> The method according to any one of <1> to <4>, wherein the non-human animal is a rodent.
<6> The method according to any one of <1> to <5>, wherein a blood vessel into which the tumor cell is injected is a saphenous artery.
<7> an animal model of osteoblastic bone metastasis, which is a non-human animal and forms an osteoblastic lesion by administering a calcineurin inhibitor to the non-human animal and injecting a tumor cell into an artery or a vein of the non-human animal, wherein the non-human animal and the tumor cell are in an allogeneic or xenogeneic relation.
<8> The animal model of osteoblastic bone metastasis according to <7>, wherein the tumor cell is a prostate cancer cell or a breast cancer cell.
<9> The animal model of osteoblastic bone metastasis according to <7> or <8>, wherein the non-human animal is a rodent.
<10> The animal model of trsteoblastic bone metastasis according to any one of <7> to <9>, wherein a blood vessel into which the tumor cell is injected is a saphenous artery.

### [Advantageous Effects of Invention]

The present invention makes it possible to form an osteoblastic lesion in a non-human animal with quite a high probability of 100%. Moreover, the osteoblastic lesion is to be formed in a wide range. Further, tracing or controlling the blood flow of the artery or the vein injected with the tumor cells also makes it possible to form an osteoblastic lesion in a desired bone. Hence, the uses of the production method of the present invention and an animal model of osteoblastic bone metastasis produced by the method also enable a highly efficient and sensitive screening for compounds useful in treating, preventing, or diagnosing osteoblastic bone metastases.

### [Brief Description of Drawings]

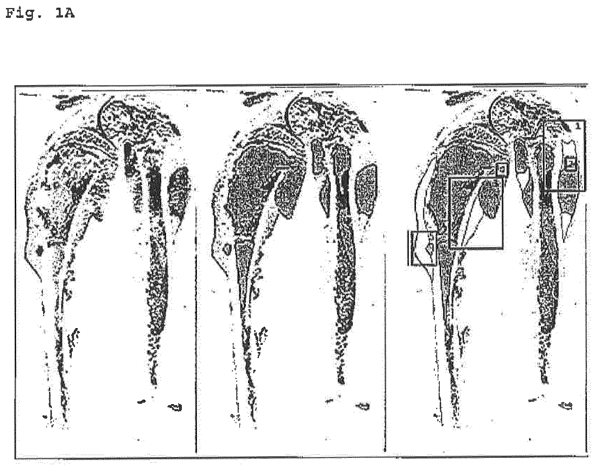

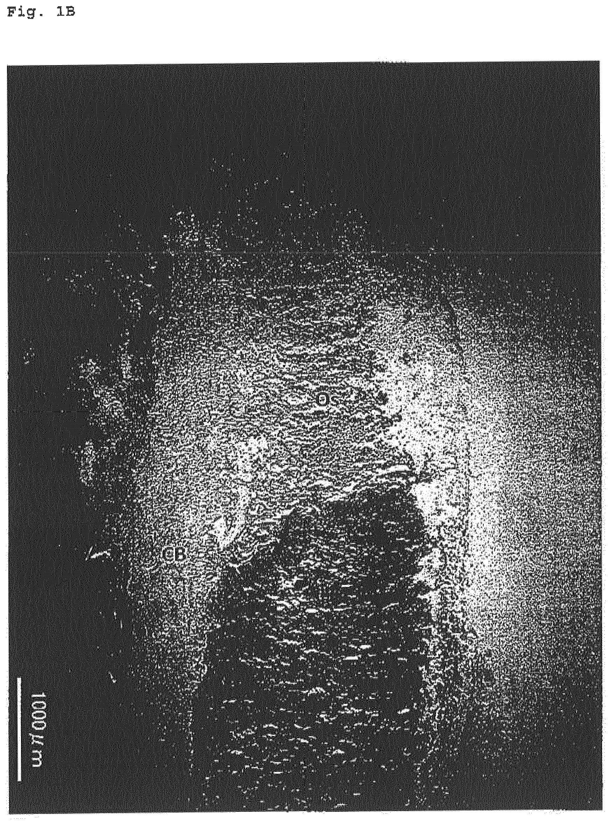

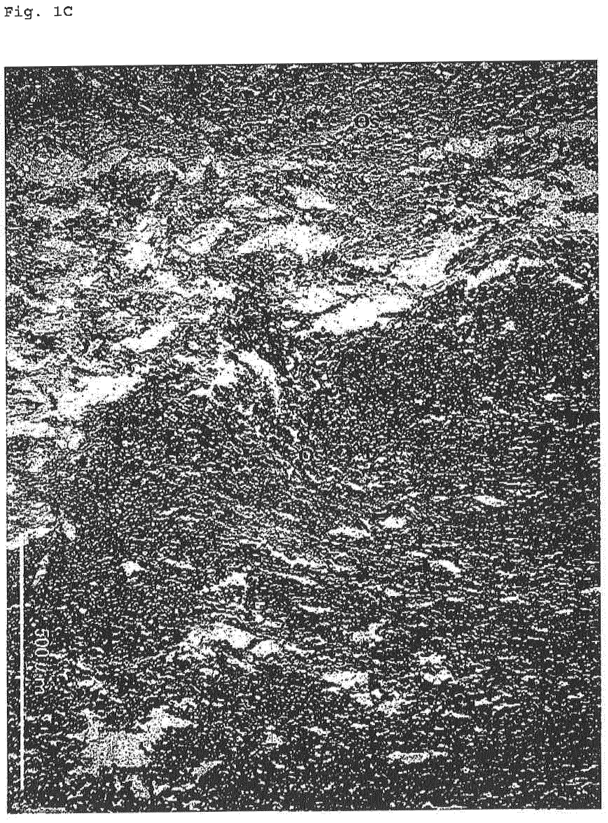

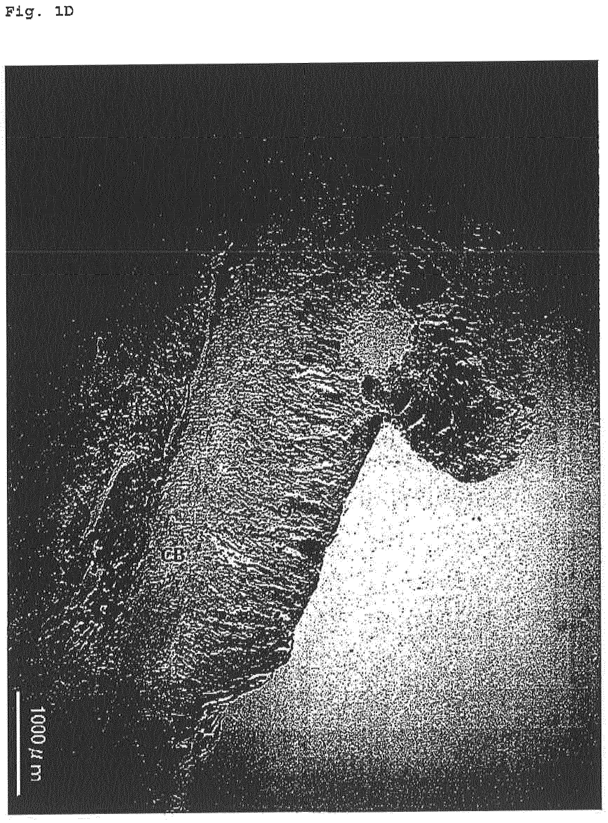

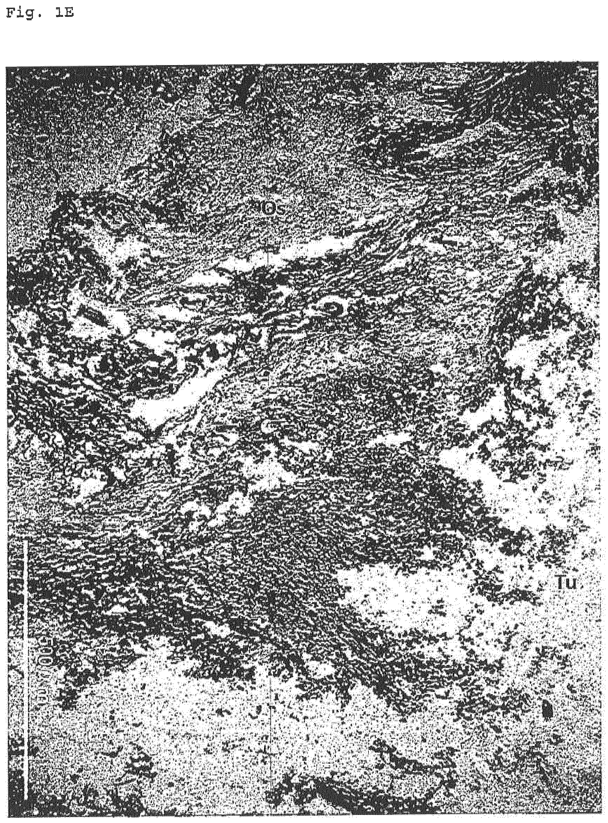

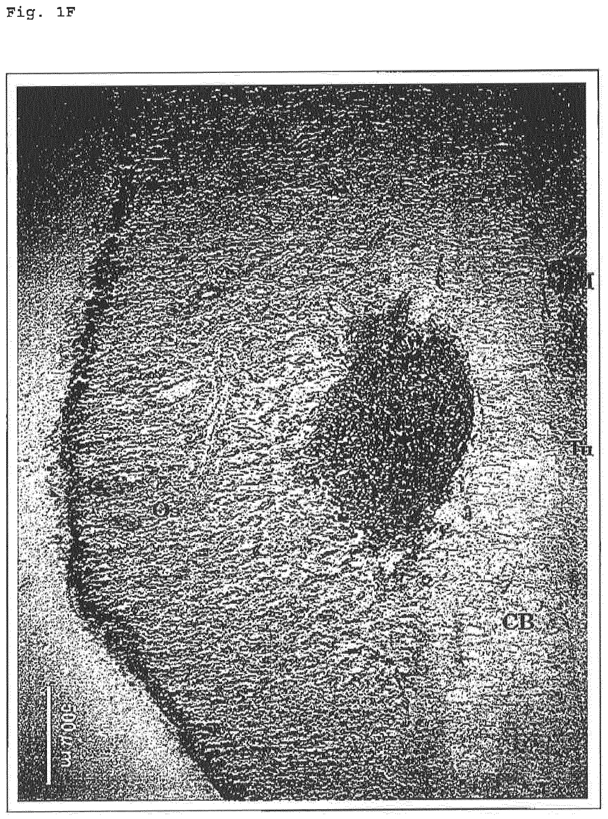

[Fig. 1A] Fig. 1A shows photographs for illustrating the result of the microscope observation of toluidine-blue stained cross sections of lower limb bones (femur and tibia) of a SD rat after ciclosporin administration and injection of rat prostate cancer cells (AT6-1) into a saphenous artery of the rat. Sites where AT6-1 metastasized (gray areas in the black and white display, or aqua color areas in the color display) are shown in the photograph in the middle of the figure. Further, osteoblastic lesion sites (white areas in the black and white display, or yellow areas in the color display) are shown in the photograph on the right.
[Fig. 1B] Fig. 1B is a micrograph for illustrating the result of enlarging and observing a site (1) surrounded by a square in the photograph on the right in Fig. 1A. Moreover, "BM" indicates an area of bone marrow, "CB" indicates an area of cortical bone, "Os" indicates an area of osteoid, and "Tu" indicates an area of a site where AT6-1 metastasized (hereinafter, regarding the representations in the figure, the same shall apply also to Figs. 1C to 1F, 5B, and 5C).
[Fig. 1C] Fig. 1C is a micrograph for illustrating the result of enlarging and observing a site (2) surrounded by a square in the photograph on the right in Fig. 1A.
[Fig. 1D] Fig. 1D is a micrograph for illustrating the result of enlarging and observing a site (3) surrounded by a square in the photograph on the right in Fig. 1A.
[Fig. 1E] Fig. 1E is a micrograph for illustrating the result of enlarging and observing a site (4) surrounded by a square in the photograph on the right in Fig. 1A.
[Fig. 1F] Fig. 1F is a micrograph for illustrating the result of enlarging and observing a site (5) surrounded by a square in the photograph on the right in Fig. 1A.
[Fig. 2] Fig. 2 shows photographs for illustrating the result of the microscope observation of toluidine-blue stained cross sections of the lower limb bones of the SD rat after the ciclosporin administration and the AT6-1 injection into the saphenous artery (left in the figure) and those of a Copenhagen rat after the AT6-1 injection into a saphenous artery thereof without the ciclosporin administration (right in the figure). Note that the representations in the figure are the same as those in Fig. 1A.
[Fig. 3A] Fig. 3A is a photograph for illustrating the result of the microscope observation of toluidine-blue stained cross sections of lower limb bones of a nude rat after the AT6-1 injection into a saphenous artery thereof. Note that the representations in the figure are the same as those in Fig. 1A.
[Fig. 3B] Fig. 3B is a micrograph for illustrating the result of enlarging and observing a site surrounded by the larger square in Fig. 3A. Note that the representations in the figure are the same as those in Fig. 1B.
[Fig. 3C] Fig. 3C is a micrograph for illustrating the result of enlarging and observing a site surrounded by the smaller square in Fig. 3A. Note that the representations in the figure are the same as those in Fig. 1B.
[Fig. 4] Fig. 4 illustrates the result of the CT analysis of a lower limb (transplanted limb) of a SD rat after the ciclosporin administration and injection of rat prostate cancer cells (AT6-1) into a saphenous artery of the rat. In the figure, the arrows indicate sites of osteoblastic lesions, and triangles indicate sites where AT6-1 metastasized.

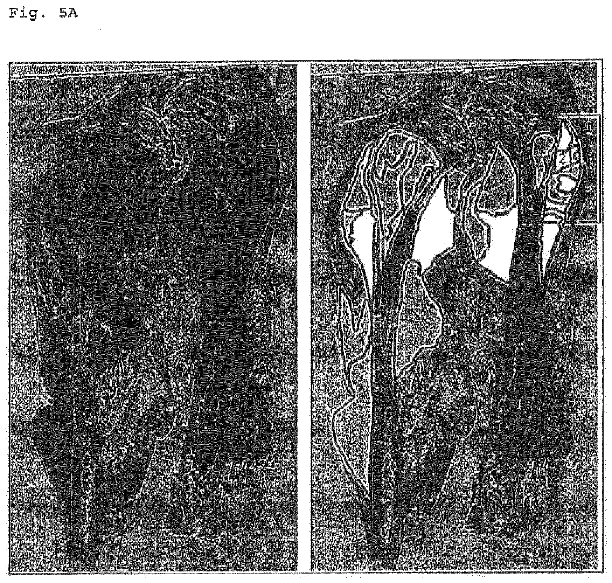

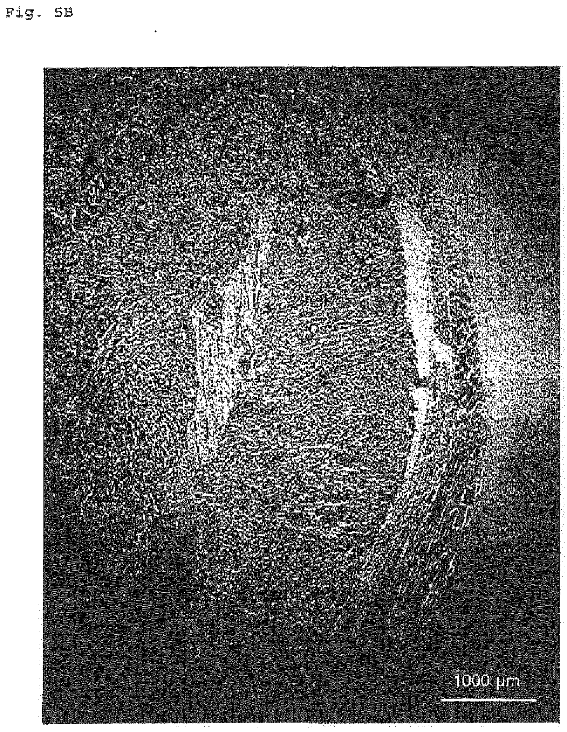

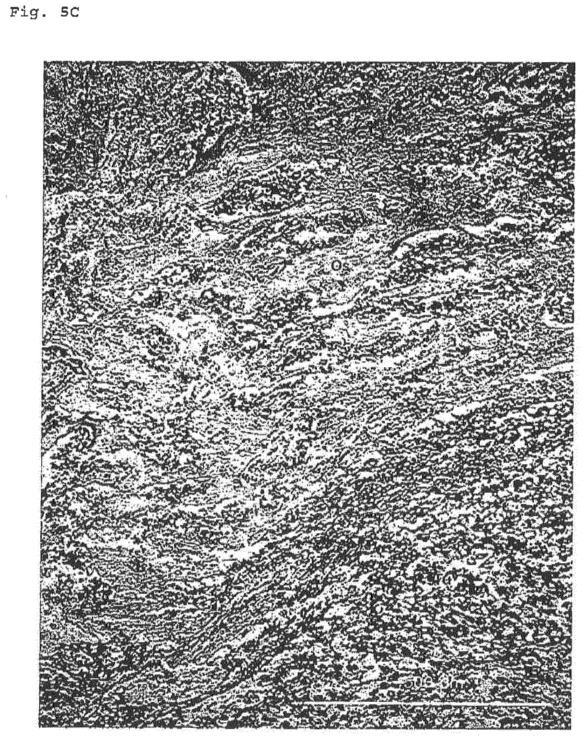

[Fig. 5A] Fig. 5A shows photographs for illustrating the result of the microscope observation of toluidine-blue stained cross sections of lower limb bones (femur and tibia) of a SD rat after the ciclosporin administration and injection of rat breast cancer cells (MRMT-1) into a saphenous artery of the rat. Sites where MRMT-1 metastasized (gray areas in the black and white display, or aqua color areas in the color display) are shown in the photograph on the right in the figure. Further, osteoblastic lesion sites (white areas in the black and white display, or yellow areas in the color display) are shown.
[Fig. 5B] Fig. 5B is a micrograph for illustrating the result of enlarging and observing a site (1) surrounded by a square in the photograph on the right in Fig. 5A.
[Fig. 5C] Fig. 5C is a micrograph for illustrating the result of enlarging and observing a site (2) surrounded by a square in the photograph on the right in Fig. 5A.

### [Description of Embodiments]

### <Model Animal for Osteoblastic Bone Metastasis, and Method for Producing the Model Animal>

A method for producing an animal model of osteoblastic bone metastasis of the present invention is a method comprising the steps of:
administering a calcineurin inhibitor to a non-human animal; and
injecting a tumor cell into an artery or a vein of the non-human animal, wherein
the non-human animal and the tumor cell are in an allogeneic or xenogeneic relation.

In the present invention, the term "osteoblastic bone metastasis" means that when a tumor cell is injected into a non-human animal, at least one site where bone formation is promoted (osteoblastic lesion) is formed in the non-human animal as a result of the action of the tumor cell on a bone tissue. To be more specific, the bone metastasis in the present invention includes not only osteoblastic bone metastases (osteoblastic dominance), but also mixed bone metastases from which an osteolytic lesion or the like is also observed. Moreover, the bone metastasis in the present invention also includes osteolytic bone metastases (osteolytic dominance), as long as an osteoblastic lesion is also formed.

In the present invention, the "non-human animal" used for the osteoblastic bone metastasis model preparation is not particularly limited. Examples thereof include vertebrates such as mammals including mice, rats, hamsters, guinea pigs, rabbits, pigs, miniature pigs, sheep, cats, dogs, and the like. Any mammals other than human can be used. The sex, age, and so forth are not particularly limited. Moreover, the "non-human animal" according to the present invention is preferably rodents such as mice, rats, hamsters, and guinea pigs, more preferably rats, and furthermore preferably a Sprague-Dawley (SD) rat or a Wister rat. Furthermore, as described later in Examples, the "non-human animal" according to the present invention is preferably a non-human animal whose immune functions (such as the function of T cells) are not suppressed.

In the present invention, the "calcineurin inhibitor" administered before or during the injection of the tumor cell means a compound having an activity of suppressing the enzyme activity of calcineurin. Examples of the "calcineurin inhibitor" include ciclosporin and tacrolimus. Preferable is ciclosporin. Moreover, it is known that suppressing the calcineurin activity suppresses the IL-2 production. Accordingly, the compound may be a compound having an activity of suppressing the IL-2 production. Note that "suppress" and related terms in the present invention include not only partial suppression but also complete suppression (inhibition).

Further, the method for administering a calcineurin inhibitor to a non-human animal is not particularly limited. Examples thereof include oral administration, subcutaneous administration, intravenous administration, intraarterial administration, intraperitoneal administration, intradermal administration, tracheobronchial administration, rectal administration, and intramuscular administration.

In addition, the calcineurin inhibitor is administered desirably before the tumor cell is injected from the viewpoint of suppressing the enzyme activity of calcineurin before the injection. Nevertheless, the calcineurin inhibitor may be administered simultaneously with the injection of the tumor cell. Further, the calcineurin inhibitor may be administered once or multiple times, or may be administered persistently by utilizing a drug delivery system (osmotic pump) or the like. Moreover, the calcineurin inhibitor may be administered continuously even after the injection of the tumor cell to be described later. Further, from the viewpoint of ease of operation, it is preferable to persistently and subcutaneously administer the calcineurin inhibitor to a non-human animal. Meanwhile, those skilled in the art can adjust the amount of the calcineurin inhibitor administered as appropriate in accordance with the kind, body weight, age, and so forth of the target non-human animal. Nevertheless, the amount is normally 1 to 150 mg/kg body weight, preferably 2 to 50 mg/kg body weight. Such an amount of the calcineurin inhibitor may be administered once or multiple times separately (for example, every day for 2 weeks).

In the present invention, the tumor cell injected into the non-human animal after or during the administration of the calcineurin inhibitor should have an activity of promoting bone formation in a bone tissue. Examples of the tumor cell include cells of prostate cancer, breast cancer, gastrointestinal cancer (such as undifferentiated stomach cancer), and ovarian cancer. From the fact that osteoblastic bone metastases occur frequently in cancer patients, preferable is a prostate cancer cell or a breast cancer cell, and more preferable are a rat prostate cancer cell or a rat breast cancer cell. Among rat prostate cancer cells, R-3327 or lines derived therefrom are further preferable, and AT6-1 is particularly preferable. Among rat breast cancer cells, MRMT-1 is further preferable. Note that the tumor cell according to the present invention has to be in an allogeneic or xenogeneic relation to the non-human animal injected with the tumor cell.

In the present invention, those skilled in the art can adjust the number of tumor cells injected as appropriate in accordance with the kind, body weight, age, and so forth of the target non-human animal. Nevertheless, the number per injection site is normally 1.0×10⁴ to 1.0×10⁷, preferably 1.0×10⁴ to 5.0×10⁵, and more preferably 2.5×10⁴ to 2.0×10⁵.

Moreover, the tumor cells are normally suspended in a solution and injected by an injection. Such a solution should be suited for the survival of the cell. Examples of the solution include buffer solutions (such as HBSS, saline, PBS, HEPES) and media (such as RPMI 1640, DMEM, MEM).

Further, the timing of injecting the tumor cell should be after or during the ciclosporin administration (in the case of the sustained administration, after or when the administration is started). Those skilled in the art can adjust the timing as appropriate in accordance with the kind, body weight, age, and so forth of the target non-human animal. Nevertheless, the timing is normally on Days 1 to 5, preferably on Days 2 to 5. In addition, the tumor cell is injected normally once, but may be injected multiple times separately.

Note that, in the present invention, "Day(s)" refer to the number of days counted without including the first day such as the day when the administration is started.

In the present invention, the tumor cell is injected from an artery or a vein. The artery or the vein from which the tumor cell is injected is not particularly limited, and depends on a bone in which an osteoblastic lesion is desirably formed. The artery or the vein suitably utilized is located on the upstream side of the bone in a blood flow direction; in other words, the artery or the vein enables a delivery to a target bone of the injected tumor cell through the blood flow. Alternatively, instead of utilizing such an artery or a vein located on the upstream side, those skilled in the art may control the blood flow by ligating surrounding blood vessels or by other means so that the tumor cell can reach a desired bone (for example, as described in Non Patent Literature 2, clipping the abdominal vein to stop the blood flow so that the tumor cell injected from the tail vein can be guided to a lumbar vertebra). Further, in a case where an osteoblastic lesion is formed in a lower limb bone, it is possible to utilize, for example, a saphenous artery, a superficial epigastric artery, a superior epigastric artery, an inferior epigastric artery, a deep femoral artery, a lateral circumflex femoral artery, a popliteal artery, or an external iliac artery. From the viewpoint of ease of operation, more preferable is the injection into a saphenous artery, a superficial epigastric artery, or a superior epigastric artery, and particularly preferable is the injection into a saphenous artery.

The production method thus described hereinabove makes it possible to form an osteoblastic lesion in a non-human animal with quite a high probability of 100% as described later in Examples. Moreover, the osteoblastic lesion is to be formed in a wide range. Further, tracing or controlling the blood flow of the artery or the vein injected with the tumor cell also makes it possible to form an osteoblastic lesion in a desired bone. Accordingly, an animal model of osteoblastic bone metastasis produced by the production method of the present invention is useful in screening for compounds useful in treating, preventing, or diagnosing osteoblastic bone metastases. Thus, the present invention also provides the following animal model of osteoblastic bone metastasis.

An animal model of osteoblastic bone metastasis, which is a non-human animal and forms an osteoblastic lesion by administering a calcineurin inhibitor to the non-human animal and injecting a tumor cell into an artery or a vein of the non-human animal, wherein the non-human animal and the tumor cell are in an allogeneic or xenogeneic relation.

Moreover, in the present invention, the time when an osteoblastic lesion is formed depends on the non-human animal used, the kind of the tumor cell injected, the number of cells, and so forth. Nevertheless, the time is normally on Days 14 to 28 after the tumor cell is injected. Moreover, the range of the osteoblastic lesion sites formed as described above depends on the non-human animal used, the kind of the tumor cell, and so forth. Nevertheless, the range is normally 0.5% or more, preferably 1% or more, and more preferably 2% or more, provided that the entire cross section of a bone in a longitudinal direction is taken as 100%. Further, the site where the osteoblastic lesion is formed is not particularly limited. The site is normally outside a bone marrow cavity, but may be inside a bone marrow cavity. Additionally, the animal model of osteoblastic bone metastasis of the present invention may simultaneously exhibit a metastasis to another organ (such as lung, lymph node, liver, kidney), in addition to a bone metastasis.

### <Screening Method for Compound for Detecting osteoblastic Bone Metastasis>

As described above, in the animal model of osteoblastic bone metastasis produced according to the present invention, an osteoblastic lesion is formed in a wide range in a desired bone with a probability of 100%. Hence, the present invention is suitably usable also in screening for a compound useful in diagnosing an osteoblastic bone metastasis.

Accordingly, the present invention also provides the following screening method for a compound for detecting an osteoblastic bone metastasis.

A screening method for a compound for detecting an osteoblastic bone metastasis, the method comprising the steps of:
(1) administering a calcineurin inhibitor to a non-human animal;
(2) injecting a tumor cell into an artery or a vein of the non-human animal to form an osteoblastic lesion;
(3) providing the non-human animal with a test compound;
(4) detecting the test compound at a site of the osteoblastic lesion; and
(5) based on an accumulated amount of the test compound detected at the site of the osteoblastic lesion, judging whether or not an osteoblastic bone metastasis is detectable with the compound, wherein
the non-human animal and the tumor cell are in an allogeneic or xenogeneic relation.

The steps (1) and (2) are as described above. The "test compound" provided to the animal model of osteoblastic bone metastasis produced by these steps is not particularly limited. Examples thereof include synthetic low-molecular-weight compounds, sugars (such as monosaccharides, disaccharides, oligosaccharides), antibodies, peptides, nucleotides, lipids, libraries constituted of the compounds, substances released by bacteria, liquid extracts and culture supernatants of cells (microorganisms, plant cells, animal cells), extracts derived from marine organisms, plants, or animals, soils, and random phage peptide display libraries. Moreover, these compounds may be labeled for the detection by a method to be described later. Examples of such a label include radioisotopes (such as ^{99m}Tc, ¹⁸F, ¹⁴C, ³H, ⁸⁹Zr, ¹¹¹In, ¹²³I, ¹²⁴I, ¹²⁵I, ¹³¹I, ⁶⁸Ga, ⁶⁴Cu, ¹⁵O, ⁹⁷Ru, ⁶⁷Cu, ¹¹C, ¹³N), paramagnetic isotopes (such as ¹⁵³Gd, ¹⁵⁷Gd, ⁵⁵Mn, ¹⁶²Dy, ⁵²Cr, ⁵⁶Fe), contrast agents (such as gadolinium, gadolinium complexes, iodine contrast agents), fluorescent substances (such as indocyanine green, IRDye800 series, fluorescein, FITC, fluorescent metals (¹⁵³Eu, lanthanide series, and the like)), chemiluminescent substances and bioluminescent substances (such as luminol, imidazole, luciferin, luciferases, GFP). The test compound can be conjugated to the label by a method known in this technical field. For example, the two may be chemically conjugated to each other directly, or may be conjugated indirectly with an appropriate linker.

The method for providing the non-human animal with the test compound is not particularly limited. Examples thereof include oral administration, subcutaneous administration, intravenous administration, intraarterial administration, intraperitoneal administration, intradermal administration, tracheobronchial administration, rectal administration, and intramuscular administration. Moreover, the concentration of the test compound provided, the number of providing performed, and the providing period are not particularly limited, and can be adjusted as appropriate in accordance with the kind and properties (such as solubility, toxicity) of the test compound.

The method for detecting the test compound is not particularly limited, either. Those skilled in the art can detect the test compound by using a known method as appropriate. Examples of such a known method include in *vivo* methods such as bioimaging techniques (scintigraphy, single-photon emission computed tomography (SPECT), positron emission tomography (PET), computerized axial tomographies (CAT, CT), PET/CT, and magnetic resonance imaging (MRI)). Moreover, the examples include *in* vitro methods such as scintigraphy, fluorescence microscope observation, mass spectrometry, and liquid chromatography analysis.

In addition, in the present invention, based on an accumulated amount of the test compound thus detected at the site of the osteoblastic lesion, it is possible to judge whether or not an osteoblastic bone metastasis is detectable with the compound. Examples of the "accumulated amount" include signal intensities (such as radiation level, fluorescence intensity, luminescence intensity) from the test compound, and the amount of the test compound, which are detected at the site of the osteoblastic lesion. Such a judging method is not particularly limited. For example, if an accumulated amount of the test compound in a bone with the osteoblastic lesion formed by the method of the present invention is significantly higher than that in a bone not subjected to osteoblastic lesion formation, the test compound can be determined to be a compound with which an osteoblastic bone metastasis is detectable.

### <Screening Method for Compound Having Activity of Suppressing Osteoblastic Bone Metastasis>

As described above, since an osteoblastic lesion is formed in a wide range in a desired bone with a probability of 100% in the animal model of osteoblastic bone metastasis produced according to the present invention, the present invention is suitably usable also in screening for a compound useful in treating or preventing an osteoblastic bone metastasis.

Accordingly, the present invention also provides the following screening method for a compound having an activity of suppressing an osteoblastic bone metastasis.

A screening method for a compound having an activity of suppressing an osteoblastic bone metastasis, the method comprising the steps of:
(1) administering a calcineurin inhibitor to a non-human animal;
(2) injecting a tumor cell into an artery or a vein of the non-human animal;
(3) providing the non-human animal with a test compound before, during, or after the injection of the tumor cell;
(4) detecting an osteoblastic lesion level in the non-human animal provided with the test compound;
(5) detecting an osteoblastic lesion level in the non-human animal injected with the tumor cell in the step (2) but not provided with the test compound; and
(6) comparing the osteoblastic lesion levels detected in the steps (4) and (5), and determining that the test compound is a compound having an activity of suppressing an osteoblastic bone metastasis if the osteoblastic lesion level detected in the step (4) is lower than the osteoblastic lesion level detected in the step (5), wherein
the non-human animal and the tumor cell are in an allogeneic or xenogeneic relation.

The steps (1) and (2) as well as the test compound and the providing method in the step (3) are as described above. The test compound may be provided before, during, or after the injection of the tumor cell. Nevertheless, in the case of screening for a compound useful in treating an osteoblastic bone metastasis, the test compound is provided to the non-human animal desirably after the injection of the tumor cell, more desirably after the osteoblastic lesion formation. Meanwhile, in the case of screening for a compound useful in preventing an osteoblastic bone metastasis, the test compound is desirably provided to the non-human animal before the injection of the tumor cell.

Moreover, examples of "detecting an osteoblastic lesion level" in the non-human animal after such steps include, as described later in Examples, bone morphology observations by microscope observation, PET, CAT, CT, PET/CT, microfocus X-ray CT (µCT), scintigraphy, SPECT, soft X-ray photographing, MRI, and the like. Additionally, the detection is possible also by detecting a bone formation marker (bone alkaline phosphatase (BAP), osteocalcin (OC), procollagen type I C-terminal propeptide (PICP), procollagen type I N-terminal propeptide (PNCP)). In addition, examples of the method for detecting such a marker include immunohistochemical analysis, CLEIA, EIA, and ELISA. Further, the osteoblastic lesion level may be detected by detecting a symptom (such as movement disorder, bone fracture) caused by an osteoblastic bone metastasis.

Then, the osteoblastic lesion level thus detected is compared with that of a non-human animal not provided with the test compound. If the former is lower, it is possible to determine that the test substance is a compound having an activity of suppressing an osteoblastic bone metastasis.

### [Examples]

Hereinafter, the present invention will be described more specifically based on Examples and Comparative Examples. However, the present invention is not limited to the following Examples.

The present inventors made efforts to prepare animal models of osteoblastic bone metastasis by four methods described below.

### (Example 1)

First, ciclosporin was administered to Sprague-Dawley (SD) rats. Then, on Day 5 after the administration was started, rat prostate cancer cells were injected into saphenous arteries of the SD rats.

### (Comparative Example 1)

Efforts were made to prepare animal models of osteoblastic bone metastasis by the method according to Example 1, except that Copenhagen rats were used in place of the SD rats, and that no ciclosporin was administered to the Copenhagen rats.

### (Comparative Example 2)

Efforts were made to prepare animal models of osteoblastic bone metastasis by the method according to Example 1, except that Copenhagen rats were used in place of the SD rats, and that the cancer cells were injected into the Copenhagen rats on Day 4 after the ciclosporin administration was started.

### (Comparative Example 3)

Efforts were made to prepare animal models of osteoblastic bone metastasis by the method according to Example 1, except that F344 nude rats (T-cell-function deficient rats) were used in place of the SD rats, and that no ciclosporin was administered to the nude rats.

In addition, the details of the rats, cells, and experimental methods used in these four preparation methods were as follows.

### <Rats and Cells>

The SD rats were provided from Japan SLC, Inc., and three 9-week-old males were used. The Copenhagen rats were provided from Japan SLC, Inc. ; two 10-week-old males and three 13-week-old males, five in total, were used in Comparative Example 1; two 17-week-old males were used in Comparative Example 2. The F344 nude rats were provided from CLEA Japan, Inc., and two 19-week-old males were used.

Moreover, the prostate cancer cells injected into these rats were a subline AT6-1 of the prostate cancer cell line R-3327 derived from Copenhagen rat. To put it differently, AT6-1 and the rats injected therewith were in an allogeneic relation in Example 1 and Comparative Example 3, and in a syngeneic relation in Comparative Examples 1 and 2.

### <Ciclosporin Administration>

Into each of the rats, an osmotic pump was subcutaneously implanted as follows, and ciclosporin was persistently administered.

First, an osmotic pump (ALZET (registered trademark) Osmotic Pump, Model: 2ML4) and a flow moderator attached thereto were measured for the empty weights. Next, a filling tube was attached to a 2-cc syringe (manufactured by Terumo Corporation) having approximately 2 mL of a ciclosporin solution (50 mg/mL, product name: SANDIMMUN(registered trademark) intravenous drip 250 mg manufactured by Novartis Pharmaceuticals Corporation) sucked therein. Then, bubbles inside the syringe were removed. Subsequently, the osmotic pump was perpendicularly held with an insertion opening thereof facing upward. The filling tube was inserted into the insertion opening to a position where the filling tube no longer moved. Thereafter, the plunger of the syringe was slowly pushed to inject the ciclosporin solution into the osmotic pump. After the leakage of the solution from the insertion opening was observed, the injection was stopped, and the filling tube was pulled out. The leaked solution was wiped off with KimWipes or the like, and then the flow moderator was inserted into the osmotic pump from the insertion opening and pushed therein until the flange reached the pump. The leaked solution was wiped off with KimWipes or the like again. Subsequently, the weight of the osmotic pump to which the flow moderator was attached was measured. Based on the empty weights, the weight (volume) of the injected ciclosporin solution was calculated. Note that when the calculated volume was 90% or more of the fill volume of the osmotic pump, it was considered as acceptable because the possibility of bubble inclusion was very low. Thus, such an osmotic pump was implanted into the rat as follows.

After the osmotic pump was filled with the ciclosporin solution as described above, the rat was anesthetized with isoflurane and held in the prone position, and the back surface from the neck to the hip was shaved. Approximately 1 cm of the skin at the back and the hip was cut with scissors. The osmotic pump was inserted into the cut area, and the skin was then sutured with a 5-0 surgical suture. Subsequently, 10 µL of an antibiotic (fosfomin S) was subcutaneously injected. The rat was returned to a cage and raised. Note that, in Comparative Examples 1 and 3, the following prostate cancer cells were injected into the rats without performing the above-described osmotic pump implantation surgery.

### <Prostate Cancer Cell Injection>

On Day 4 or 5 after the ciclosporin administration was started, or without the ciclosporin administration, the prostate cancer cells (AT6-1) were injected into a saphenous artery, which is a femoral artery branch, of the rat as follows.

First, AT6-1 was treated with trypsin and separated from the culture dish, and the number of cells was counted. The cells were suspended in a Ca²⁺/Mg²⁺-free Hanks' balanced salt solution (HBSS) at a concentration of 2×10⁶ cells/mL. The suspension was ice cooled until injection into the rat.

Moreover, the rat was anesthetized with isoflurane and held in the supine position. Then, the hind legs and the groin were shaved and disinfected with 70% ethanol/povidone-iodine. After 0.01 to 0.02 mL (0.2 mg/kg) of Metacam 0.5% injection was subcutaneously injected, approximately 2 to 3 cm of the skin at the groin was cut. Subsequently, a superficial epigastric artery, a saphenous artery, and so forth were observed, and membranes surrounding blood vessels and nerves were separated. The saphenous artery, veins, and nerves were separated independently. Thereafter, a single ligature (3-0) was passed through the saphenous artery at a distal portion of a popliteal artery bifurcation area, and tissues surrounding a superficial epigastric artery bifurcation area were separated (this ligature is also referred to as surgical suture (1)). A single ligature was passed through the femoral artery between the superficial epigastric artery bifurcation area and the popliteal artery bifurcation area (the ligated femoral artery is located a little farther from the superficial epigastric artery bifurcation area), and several drops of a smooth muscle relaxant (papaverine hydrochloride injection) were dripped along the saphenous artery (this ligature is also referred to as surgical suture (2)).

Into 29G Myjector, 0.1 mL of the cell suspension was sucked. While ends of the surgical sutures (1) and (2) were clamped with forceps, the saphenous artery was lifted to avoid the back flow of the cells and the flow from the saphenous artery into the peripheral part. A cotton swab was inserted under the saphenous artery, the needle was pierced from the distal portion of the lifted saphenous artery in the central direction, and the cell suspension was slowly injected (0.1 mL/10 seconds). Then, the needle was slowly pulled out. After no bleeding was confirmed, an instant adhesive (product name: Aron Alpha) was dripped to the pierced hole. A fat mass was placed thereon to close the pierced hole, and no bleeding was confirmed. Subsequently, the surgical sutures (1) and (2) were loosened, the blood flow from the femoral artery was resumed, and no bleeding was confirmed. Further, the operative field was filled with 70% ethanol and left for approximately 10 seconds to remove the tumor cells leaked outside the blood vessel. Thereafter, the 70% ethanol was sucked into absorbent cotton, and the operative field was filled with a saline, and then sufficiently washed. After the operative field was washed with a saline again, the muscles and tissues in the operative field were restored, and the skin was closed with a surgical suture (5-0).

Note that, in Example 1 and Comparative Examples 1 and 3, one hind leg was treated by injecting the cell suspension therein according to the above-described method, while the other hind leg was treated as described above, except that HBSS was injected instead of the cell suspension. Moreover, in Comparative Example 2, both hind legs were treated by injecting the cell suspension therein according to the above-described method. After such treatments, the antibiotic (fosfomin S) was subcutaneously injected, and the rats were returned to the cages and raised.

### <Toluidine Blue Staining of Bone Nonfixed Frozen Sections>

To detect osteoblastic bone metastases in the prostate-cancer injected rats, toluidine blue staining was performed as follows.

First, the femur, the tibia, and the fibula were cut around the hip joint and the ankle. Without dislocating the joint thereamong, these three bones (lower limb bones) were isolated from the rat injected with the prostate cancer as described above, and frozen sections were prepared according to the Kawamoto's film method (preparation method for nonfixed undecalcified frozen sections from hard tissues).

To be more specific, as described later, since an osteoblastic lesion might be formed on a bone surface, the lower limb bones were extracted from the rat 2 to 3 weeks after the prostate cancer was injected, so that surrounding muscles were left in small amounts. Then, the head of the femur and a distal end of the tibia were cut, the lower limb bones were bent in the form of pine leaf, and a proximal end of the femur and the cut distal end of the tibia were threaded and fixed.

Next, the lower limb bones were placed on a stainless mesh ladle (cooking tool for skimming), immersed in a cooling solution (isopentane/dry ice), and rapidly frozen (for approximately 10 seconds) while the ladle was being moved. Then, a pre-cooled embedding agent (SCEM manufactured by Section-Lab Co., Ltd.) was placed in a stainless embedding container. The coolant on the bone surfaces was wiped off, and the lower limb bones were immersed in the embedding agent. Subsequently, the entire embedding container was immersed in a cooling solution. While the embedding container was being moved, the bones were frozen (to such an extent that the upper surface of the embedding agent started freezing, within approximately 10 seconds). The embedding container was lifted such that the upper surface of the embedding agent was at the same height as the surface of the cooling solution. In this state, the embedding agent was completely frozen. The obtained embedded block was transferred into a cryostat (-20°C), and 5-µm sections were prepared using the cryostat, so that the resulting cross sections were parallel to major axes of the bones. Thereafter, the sections were left in the cryostat for 30 minutes, and freeze-dried. After that, the sections were taken out from the cryostat and dried at room temperature for 1 minute. Next, the sections were immersed in anhydrous ethanol for 3 to 5 seconds, and then unnecessary ethanol was absorbed into Kimtowel. Immediately thereafter, the sections were immersed in a 4% paraformaldehyde solution for 1 minute or longer. The sections were washed by placing the sections in and out of pure water for 10 seconds. After that, the sections were immersed in a 0.05% toluidine blue solution (pH: 7) for 5 minutes. Then, the sections were washed again by placing the sections in and out of pure water for 30 seconds. Subsequently, unnecessary water was absorbed into Kimtowel. Thereafter, several drops of a dedicated mounting agent SCMM-R3 were dripped on the tissue surface. After that, an adhesive film supporting the section was cut inside a double-sided tape of a microscope slide by using a cutter. Moreover, another new microscope slide on which several drops of SCMM-R3 were dripped was prepared, and the adhesive film supporting the section was placed on the microscope slide with the tissue surface facing downward. Two stripped filter papers were slided right and left on the adhesive film supporting the section to absorb excessive SCMM-R3. Note that, when bubbles were included between the microscope slide and the film, the film was lifted using pointed tweezers, one or two drops of SCMM-R3 were dripped, and excessive SCMM-R3 was absorbed again using filter papers. Next, the microscope slide was set in a section mounting system, irradiated with ultraviolet for 1 minute, and then washed with water.

Then, the bone nonfixed frozen section thus prepared was observed with a digital microscope (All-in-one microscope manufactured by Keyence Corporation), and staining images were obtained. Figs. 1A to 3C show representative staining images among the obtained results on Day 18 after the prostate cancer was injected.

### <Computed Tomography (CT)>

To detect osteoblastic bone metastases in the prostate-cancer injected rats, a CT analysis was also performed as follows. Specifically, on Days 15 to 20 after the prostate cancer was injected, the rats were anesthetized by introducing isoflurane thereto. In the supine position, the abdomen and the legs of each rat were immobilized on a rat holding bed using a tape and the like. Then, the rat was placed in a CT system and irradiated with X-rays. CT images of the knee joint and the surrounding of the hind legs (raw files) were obtained under the following imaging conditions.
Project count: 180 views
Frames averaged: 1 frame/view
Detector binning: 2x2
X-ray tube current: "Default (150 µA)"
X-ray tube voltage: 75 kV
Exposure time: 230 ms
Magnification: 2.6

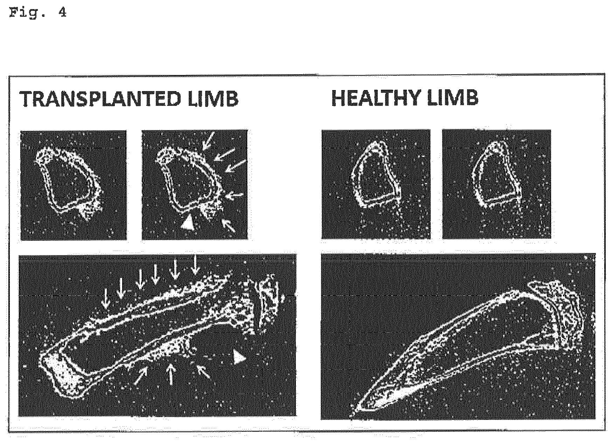

The obtained raw files were reconstructed using Trifoil Console (reconstruction condition: Half Res). The reconstructed images were further converted using image display software, and hdr and img files were created. These were read by ImageJ, and the images were displayed for the analysis. Fig. 4 shows the obtained result.

As shown in Figs. 1A to 1F, in the rats prepared by the method according to Example 1, the metastases of the tumor cells were observed on the tibia surface and inside the bone marrow cavity approximately 2 to 3 weeks after the cells were injected (in Figs. 1A to 1F, gray areas in the black and white display, or aqua color areas in the color display). Further, it was revealed that osteoblastic lesions were formed in a wide range outside the bone marrow cavity at a proximal portion of the tibia or a distal portion of the femur (near the knee). Further, it was revealed as shown in Fig. 4 that, in the rats prepared by the method according to Example 1, the osteoblastic lesions were detectable also by the CT analysis. Moreover, osteoblastic lesion formations in such a wide range were observed in all the prepared rats (as a result of transplanting the tumor cells into one hind leg of each of the three rats, the three legs were positive (three legs out of three legs, 100%)). Thus, it was also revealed that the method according to Example 1 was quite an excellent method also in the efficiency of forming osteoblastic lesions.

Note that, although unillustrated, a total of five lower limbs of other three rats were treated by adopting the method according to Example 1, except that the rat prostate cancer cells were injected on Day 3 after the ciclosporin administration was started (one rat was treated by injecting the cells into only the right lower limb; two rats were treated by injecting the cells into both of right and left lower limbs). The result showed that osteoblastic lesions were formed in a wide range outside the bone marrow cavity in any of the rat lower limbs in the same manner as described above. Moreover, a radioactive pharmaceutical (technetium hydroxymethylene diphosphonate (^{99m}Tc), product name: CLEARBONE, manufactured by Nihon Medi-Physics Co., Ltd.), which would accumulate on new bone portions, was used to analyze the rats injected with these tumor cells on Day 18 (the one rat treated by injecting the cells into only the right lower limb) and on Day 22 or 23 (the two rats treated by injecting the cells into both of right and left lower limbs) after the injection, and to evaluate areas of the osteoblastic lesions where the radioactive pharmaceutical accumulated. As a result, provided that the entire cross section (the tibia and the femur) was taken as 100%, the percentage of the lesion sites was 1.22 ± 0.63% (those of the lower limbs were 0.91%, 0.50%, 1.67%, 0.95%, and 2.05%).

On the other hand, in the method according to Comparative Example 1 using the Copenhagen rats with no ciclosporin administered, osteoblastic lesion formations were detected outside the bone marrow cavity in all of the prepared rats (as a result of transplanting the tumor cells into one hind leg of each of the four rats, the four legs were positive (four legs out of four legs, 100%).

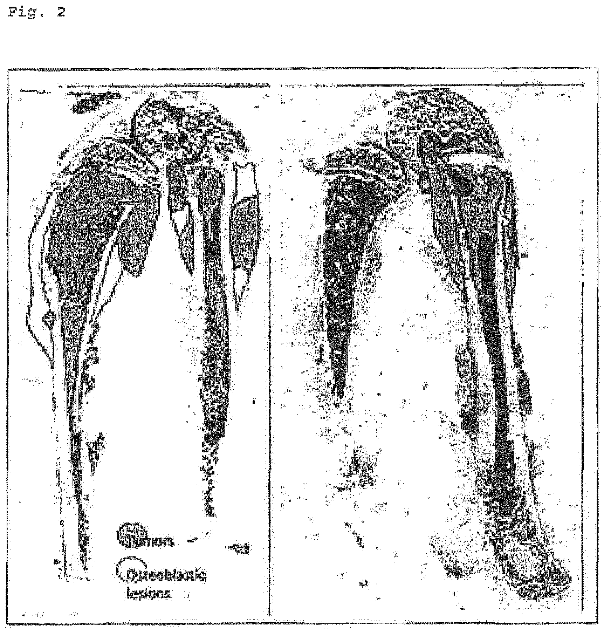

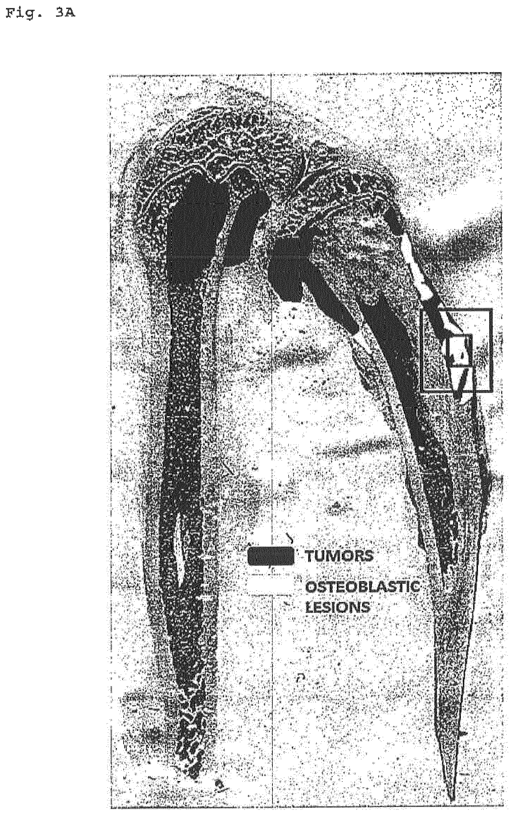

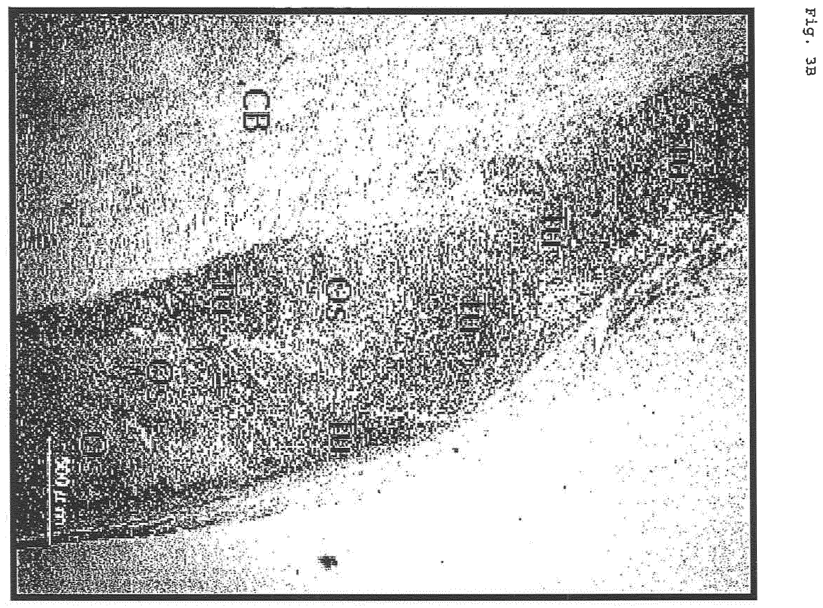

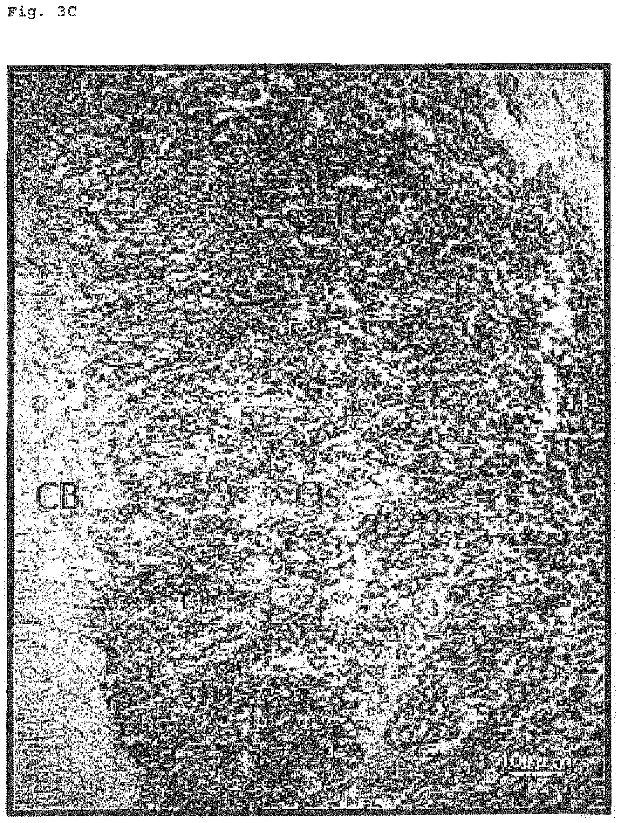

However, the sites were very small (see Fig. 2). Moreover, as a result of administering ciclosporin to two of the Copenhagen rats and transplanting the tumor cells into each of the right and left hind legs, osteoblastic lesion formations were detected outside the bone marrow cavity in only one leg (one leg out of four legs, 25%) (Comparative Example 2). The lesion sites were small as in the case of Comparative Example 1. Further, in the method according to Comparative Example 3 using the nude rats with no ciclosporin administered, osteoblastic lesion formations were detected in a relatively wide range outside the bone marrow cavity in one (50%) of the two rats into one hind leg of which the tumor cells were transplanted (see Figs. 3A to 3C). However, the sites were smaller than those of Example 1.

As described above, it was revealed that administering ciclosporin, one of calcineurin inhibitors, to rats and then injecting the arteries thereof with the tumor cells allogeneic in relation to the rats formed osteoblastic lesions in a wide range with quite a high probability of 100% (Example 1).

On the other hand, even if the syngeneic tumor cells were injected into arteries of rats, the obtained osteoblastic lesions were very small (Comparative Examples 1 and 2). Further administering ciclosporin decreased the probability of forming a lesion to 25% (Comparative Example 2).

Note that, in Non Patent Literature 2 also, efforts had been made to prepare osteoblastic bone metastasis models by injecting R-3327, which was the parental line of AT6-1, into the left ventricle of the heart or the tail vein of Copenhagen rats. R-3327 and the rats were in a syngeneic relation as in Comparative Examples 1 and 2 of the present application. However, in this method also, osteoblastic lesions which could be detected by scintigraphy were not detected. These suggest that when tumor cells and a rat injected therewith are in an allogeneic relation and the site injected with the tumor cells is a blood vessel, no osteoblastic lesion is formed, or a lesion, if formed, is so small that it is difficult to detect the lesion.

Meanwhile, it is known that calcineurin inhibitors are normally used as immunosuppressive agents. However, even when the arteries of the rats having suppressed immune functions (nude rats) as in Comparative Example 3 were injected with the tumor cells allogeneic in relation to the rats, the probability of forming a lesion was decreased to 50%. This suggests that such quite a high probability of forming an osteoblastic lesion in Example 1 is not attributable to the suppressed immune function.

Further, the present inventors confirmed by the following method that the method of the present invention enabled osteoblastic lesion formations with quite a high probability of 100%.

### (Example 2)

Efforts were made to prepare animal models of osteoblastic bone metastasis by the method according to Example 1, except that Wister rats (two 9-week-old males) were used in place of the SD rats, and that the rat prostate cancer cells (AT6-1, 2×10⁵ cells/leg) were injected into saphenous arteries of both hind legs of each Wister rat on Day 3 after the ciclosporin administration was started.

### (Example 3)

Efforts were made to prepare animal models of osteoblastic bone metastasis by the method according to Example 1, except that Wister rats (two 9-week-old males) were used in place of the SD rats, and that rat breast cancer cells (MRMT-1, 2.5×10⁴ cells/leg) were injected into saphenous arteries of both hind legs of each Wister rat on Day 3 after the ciclosporin administration was started.

Note that the Wister rats were provided from Japan SLC, Inc. MRMT-1 was a breast cancer cell line derived from SD rat. Accordingly, in Examples 2 and 3 also, the tumor cells and the rats injected therewith were in an allogeneic relation.

The lower limbs (transplanted legs) of the rats prepared as described above were analyzed by the above-described CT on Day 17 after the tumor cells were injected. Further, in Example 2, the rats (two) were analyzed by the above-described toluidine blue staining on Day 19 after the tumor cells were injected; in Example 3, one rat was analyzed on Day 19 after the tumor cells were injected, and the other rat was analyzed on Day 21 after the tumor cells were injected.

As a result, although unillustrated, both of the analysis methods confirmed that osteoblastic lesions were formed in a wide range with a probability of 100% in Example 2.

Similarly, it was confirmed in Example 3 also by both of the analysis methods that osteoblastic lesions were formed in a wide range with a probability of 100%. More specifically, as shown in representative staining images of Example 3 (Figs. 5A to 5C), approximately 2 to 3 weeks after the tumor cells were injected, the metastases were observed on the tibia surface and inside the bone marrow cavity from a distal portion of the femur and a proximal portion of the tibia (near the knee) toward a lower portion of the lower limb. Further, osteoblastic bone lesions were detected in a wide range outside the bone marrow cavity at the distal portion of the femur (near the knee) and the proximal portion of the tibia. In addition, it was also verified that the method of the present invention enabled productions of animal models ofosteoblastic bone metastasis with quite a high probability by injecting not only the prostate cancer cells but also the breast cancer cells as described above.

### [Industrial Applicability]

As described above, the present invention makes it possible to form an osteoblastic lesion in a non-human animal with quite a high probability of 100%. Moreover, the osteoblastic lesion is to be formed in a wide range. Further, following or controlling the blood flow of the artery or the vein injected with the tumor cells also makes it possible to form an osteoblastic lesion in a desired bone.

Accordingly, the production method of the present invention and an animal model of osteoblastic bone metastasis produced by the method are useful in screening for compounds useful in treating, preventing, or diagnosing osteoblastic bone metastases.

Provided is a method for producing an animal model of osteoblastic bone metastasis. A non-human animal in which an osteoblastic lesion is formed in a wide range has been successfully produced with a probability of 100% by: administering a calcineurin inhibitor to a non-human animal; and injecting a tumor cell into an artery or a vein of the non-human animal, wherein the non-human animal and the tumor cell are in an allogeneic or xenogeneic relation.

## Claims

1. A method for producing an animal model of osteoblastic bone metastasis, the method comprising the steps of:
administering a calcineurin inhibitor to a non-human animal; and
injecting a tumor cell into an artery or a vein of the non-human animal, wherein
the non-human animal and the tumor cell are in an allogeneic or xenogeneic relation

2. A screening method for a compound for detecting an osteoblastic bone metastasis, the method comprising the steps of:
(1) administering a calcineurin inhibitor to a non-human animal;
(2) injecting a tumor cell into an artery or a vein of the non-human animal to form an osteoblastic lesion;
(3) providing the non-human animal with a test compound;
(4) detecting the test compound at a site of the osteoblastic lesion; and
(5) based on an accumulated amount of the test compound detected at the site of the osteoblastic lesion, judging whether or not an osteoblastic bone metastasis is detectable with the compound, wherein
the non-human animal and the tumor cell are in an allogeneic or xenogeneic relation.

3. A screening method for a compound having an activity of suppressing an osteoblastic bone metastasis, the method comprising the steps of:
(1) administering a calcineurin inhibitor to a non-human animal;
(2) injecting a tumor cell into an artery or a vein of the non-human animal;
(3) providing the non-human animal with a test compound before, during, or after the injection of the tumor cell;
(4) detecting an osteoblastic lesion level in the non-human animal provided with the test compound;
(5) detecting an osteoblastic lesion level in the non-human animal injected with the tumor cell in the step (2) but not provided with the test compound; and
(6) comparing the osteoblastic lesion levels detected in the steps (4) and (5), and determining that the test compound is a compound having an activity of suppressing an osteoblastic bone metastasis if the osteoblastic lesion level detected in the step (4) is lower than the osteoblastic lesion level detected in the step (5), wherein
the non-human animal and the tumor cell are in an allogeneic or xenogeneic relation.

4. The method according to any one of claims 1 to 3, wherein the tumor cell is a prostate cancer cell or a breast cancer cell.

5. The method according to any one of claims 1 to 4, wherein the non-human animal is a rodent.

6. The method according to any one of claims 1 to 5, wherein a blood vessel into which the tumor cell is injected is a saphenous artery.

7. An animal model of osteoblastic bone metastasis, which is a non-human animal and forms an osteoblastic lesion by administering a calcineurin inhibitor to the non-human animal and injecting a tumor cell into an artery or a vein of the non-human animal, wherein the non-human animal and the tumor cell are in an allogeneic or xenogeneic relation.

8. The animal model of osteoblastic bone metastasis according to claim 7, wherein the tumor cell is a prostate cancer cell or a breast cancer cell.

9. The animal model of osteoblastic bone metastasis according to claim 7 or 8, wherein the non-human animal is a rodent.

10. The animal model of osteoblastic bone metastasis according to any one of claims 7 to 9, wherein a blood vessel into which the tumor cell is injected is a saphenous artery.
